# EUROPEAN PATENT APPLICATION

(11) **EP 2 500 000 A1**
(43) Date of publication of application: **19.09.2012**
(21) Application number: 12159085.5
(22) Date of filing: 12.03.2012
(51) Int. Cl.: A61F 2/76

(54) **A passive prosthetic limb and process for realising the passive prosthetic limb**

(30) Priority: 15.03.2011 IT MI20110415
(71) Applicant: Cremonesi, Nicoletta, 28100 Novara (IT); Audenino, Alberto, 10028 Trofarello (TO) (IT)
(72) Inventor: Cremonesi, Nicoletta, 28100 Novara (IT); Audenino, Alberto, 10028 Trofarello (TO) (IT)
(74) Representative: Sutto, Luca

(57) **Abstract**

A passive artificial limb (1), comprising: a socket (3) arranged at a first end (4) of the prosthetic limb (1) and exhibiting a shape designed to abut a stump (5) of a subject's limb, a terminal part (7), optionally shaped as a hand or a foot, arranged at a second end (8) of the passive prosthetic limb (1) opposite the first end (4), a connecting part (6) interposed between the socket (3) and the terminal part (7); the connecting part (6) comprising at least an adjusting device (10) configured such as to vary at least an moment of inertia of the passive prosthetic limb (1) with respect to at least a reference axis.

## Description

### FIELD OF THE INVENTION

The invention relates to a passive artificial limb for individuals subject to amputation or affected by malformations, for example amelia, ectromelia, hemimelia and peromelia. The artificial limb is used in the medical field for replacing a part of the body that is missing. The invention further relates to a process for manufacturing the artificial limb.

### THE STATE OF THE ART

Prostheses are components suitable for partly or completely replacing a segment of the human body that is missing due to traumatic or congenital causes and events.

External prostheses are described in the following, which have the task of replacing the morphology and in part the functionality of the amputated limb.

In prostheses, the primary purpose of those relating to the lower limbs is to enable the amputee to resume walking. These prostheses can be further classified into traditional (or passive), characterized by a rigid supporting structure and hinge elements, and modular (or active), consisting of mechanical components and/or electronic components that allow a certain degree of articulation between the parts forming the prosthesis; this enables them to reproduce the various stages of the step in ambulation.

Upper limb prostheses can be instead classified according to their structural and functional characteristics, and are sub-divided into passive and active prostheses.

The goal of active or functional prostheses is to help the wearer, who is usually bilateral, to make some of the movements necessary for the performance of primary functions (feeding, personal hygiene, etc.). These prostheses may include gripping devices and/or mechanisms that enable the opening and closing of the fingers. The movement can be achieved with hooks (or manipulators), with plier systems (three-fingered, or closure of the thumb on an index-middle finger pair that are solidly constrained to one another), or with complete articulation systems of the fingers. The increased movements and constructive complexity of the prosthesis are related to the different degree of motorization desired therefor.

Passive prostheses, differently, have as their goal the reconstruction of a mutilated body segment with a view to restoring bodily integrity, with particular attention to the aesthetic aspect. These prostheses, usually suitable for wearers having a monolateral pathology, can be aid and completion elements to the patient's body and can aid passive gripping. The present invention relates in particular to this second type of prosthesis and therefore concerns a passive artificial limb.

In addition to the general overview described in the foregoing, some solutions already known in the art are now described.

A first example, described in document US5549712, relates to a passive prosthetic forearm equipped with a device for assisting the movement of the prosthesis in relation to the stump. The device can detect the angle between the stump and the prosthetic forearm and, proportionally to this angle, can exert a moment around the elbow.

A second example, described in document GB2087727, discloses a passive prosthesis for the replacement of a subject's limb. The prosthesis comprises a connecting part to the amputee's stump, an elongate intermediate bar and a gripping element positioned on the opposite end with respect to the stump. The gripping element is for example so shaped as to simulate a hand or a prosthetic foot. The intermediate bar can be substituted so as to adapt the prosthesis to the patient. Despite the fact that the prosthesis enables the intermediate bar to be interchanged, calibrations and adjustments to suit the needs of each individual patient are extremely difficult to perform. In addition to this, the above-described prosthesis is constituted by a large number of components which have an effect on the cost of the prosthesis and complicate interventions thereon. The structure of the component parts of the prosthesis is also poorly aesthetic, this being extremely important feature with respect to cosmetic artificial limbs.

A third example concerns a passive artificial limb comprising a socket suitable for abutting the stump, an intermediate part and a terminal part opposite the stump. The connecting portion is constituted for example by a prosthetic forearm, while the terminal part is constituted for example by a hand-shaped structure. The artificial limb has an aesthetic function, substantially reproduces the missing part of the subject and consists of a single body made of a solid wood or a plastic material. Optionally, an aesthetic glove made of plastic material or silicone is fitted on the prosthesis structure such as to impart a greater degree of aesthetic finish and similarity with the natural limb. The described passive artificial limb has a high overall weight that also causes a high degree of movement when applied to the connection point between prosthesis and the stump. The considerable weight and high inertia of the prosthesis hinder the patient's rehabilitation process and cause postural imbalances. Further, the artificial limb is incapable of adapting to the needs of the patient, particularly during periods of rehabilitation.

### OBJECT OF THE INVENTION

A first aim of the present invention is to obviate one or more of the drawbacks of the preceding solutions.

A further aim is to disclose a passive artificial limb that is very light and at the same time very resistant.

A further aim of the present invention is to disclose a passive artificial limb able to adapt to the needs of the subject receiving the prosthesis.

A further aim of the present invention is to disclose an artificial limb with an excellent versatility of use and which enables actively supporting the rehabilitation process of the patient.

A further aim of the present invention is to provide an artificial limb able to maintain the subject's posture unaltered.

A further aim of the invention is to make available a limb having an excellent similarity to a natural limb.

A further aim of the invention is to guarantee good passive functioning to the subject, such as for example in resting objects and levering objects.

One or more of the mentioned aims are substantially attained by a passive artificial limb according to one or more of the appended claims.

Aspects of the invention are described herein below.

A first aspect relates to a passive artificial limb, comprising: at least a socket arranged at a first end of the prosthetic limb and exhibiting a shape designed to abut a stump of a subject's limb, at least a terminal part, optionally shaped as a hand or a foot, arranged at a second end of the passive prosthetic limb opposite the first end, at least a connecting part interposed between the socket and the terminal part.

In a 2nd aspect according to the 1 st aspect, the connecting part comprises at least an adjusting device configured such as to vary at least a moment of inertia of the passive prosthetic limb with respect to at least a reference axis.

In a 3rd aspect according to the preceding aspect, the adjusting device enables a variation of the weight and/or the position of a centre of gravity of the passive prosthetic limb.

In a 4th aspect according to the 2nd or 3rd aspect, the adjusting device enables the weight and/or the position of the centre of gravity of the connecting part to be changed.

In a 5th aspect according to any one of aspects from 2 to 4, the regulating device comprises a predetermined number of application zones, arranged on the connecting part.

In a 6th aspect according to the preceding aspect each connecting part is destined to receive one or more respective discrete bodies, each of the discrete bodies exhibiting a predetermined dimension and weight, such that addition or removal of one or more of the discrete bodies from the application zone determines a change in weight and/or at least a moment of inertia of the passive prosthetic limb.

Ina 7th aspect, according to the 5th or 6th aspects, the application zones comprise a predetermined number of housing seatings destined to contain the discrete bodies.

In an 8th aspect according to the preceding aspect, each of the discrete bodies is conformed as an insert such as to be removably insertable in at least one of the housing seatings.

In a 9th aspect according to the 7th or 8th aspect, each of the housing seatings is configured such as exclusively to contain the respective discrete body.

In a 10th aspect, according to any one of aspects from 6 to 9, the adjusting device comprises at least a set of discrete bodies that are substantially identical.

In an 11th aspect, according to any one of aspects from 6 to 10, the discrete body is a modular insert composed of at least two parts.

In a 12th aspect according to any one of claims from 6 to 11, the device comprises at least a holder insertable removably in the connecting body and predisposed to bear a plurality of discrete bodies.

In a 13th aspect according to any one of aspects from 6 to 12, the discrete body exhibits a substantially prismatic shape that is complementarily shaped to the respective seating.

In a 14th aspect according to any one of aspects from 7 to 13, the seatings exhibit a respective access opening for inserting the discrete bodies.

In a 15th aspect, according to the preceding aspect, the access opening facing towards an outside of the limb at the socket in order to enable insertion of the discrete bodies when the prosthetic limb is separated from the stump.

In a 16the aspect according to any one of aspects from 7 to 15, the seatings the seatings extend internally of the limb, in particular internally of the connecting part, remaining accessible exclusively from the respective access openings, the insertion and removal of the discrete bodies not altering the appearance of the lateral surface of the connecting part.

In a 17th aspect according to any one of the preceding aspects the connecting part exhibits one or more cavities which extend at least partially along the longitudinal development of the connecting part.

In an 18th aspect according to the preceding aspect, the cavity is a lightening cavity.

In a 19th aspect according to aspects 17 or 18, the cavity extends substantially from the socket to the terminal part.

In a 20th aspect according to any one of aspects from 17 to 19, the housing seatings are arranged in a space extending radially between the cavity and an external surface of the connecting part.

In a 21st aspect according to any one of aspects from 7 to 20, a predetermined number of the housing seatings are arranged in a circular crown exhibiting an angular spacing of less than 180°, optionally in which the angular spacing is less than 90°.

In a 22nd aspect according to any one of aspects from 7 to 21, a predetermined number of housing seatings are subsequently arranged along longitudinal lines that are substantially parallel with respect to the longitudinal development of the passive prosthetic limb.

In a 23rd aspect according to any one of the preceding aspects, the control device is configured such as to vary the position of the centre of gravity of the passive artificial limb internally of a control volume.

In a 24th aspect, according to the preceding aspect, the control volume exhibits a radial size that is at most 30% of a maximum radial size of the prosthetic limb.

In a 25th aspect, according to the 23rd or 24th aspect, the control volume exhibits an axial size that is at most 25% of the maximum axial size of the prosthetic limb.

In a 26th aspect according to any one of aspects from 23 to 25, the control volume extends between the socket and a maximum axial excursion section of the centre of gravity.

In a 27th aspect according to the preceding aspect, the maximum axial excursion section being distant from the socket by a quantity which is at least from ¼ to 1/3 of the length of the prosthetic limb.

In a 28th aspect according to any one of aspects from 7 to 27, the discrete bodies are arranged between the socket and the centre of gravity of the prosthetic limb.

In a 29th aspect according to any one of the preceding aspects, the artificial limb is a prosthesis of an upper limb.

In a 30th aspect according to any one of aspects from 1 to 28, the artificial limb is a forearm prosthesis.

In a 31st aspect according to any one of the preceding aspects, the artificial limb comprises at least a covering glove exhibiting a surface finishing that is substantially alike to the epidermal surface of the subject on which the passive artificial limb is applied; the covering glove further exhibits a chromatic appearance that is substantially similar to the chromatic aspect of the subject's skin on whom the passive artificial limb is applied.

A 32nd aspect relates to a manufacturing process of a passive artificial limb according to any one of aspects from 1 to 31, comprising: detecting a shape of the patient's stump, optionally detecting a shape of a healthy limb belonging to the patient, creating a 3D mathematical model of the prosthetic limb on the basis of the detections of point a) and/or point b), realising a real physical model of the prosthetic limb on a basis of the detection of the shape of the stump and the 3D mathematical calculation.

In a 33rd aspect according to the preceding aspect the step of detecting the shape of the patient's stump comprises realising a mould, for example in plaster of Paris, of the patient's stump.

In a 34th aspect, according to aspects 32 or 33, the step of realising the physical model comprises a step of rapid prototyping to reproduce a substantially identical shape of the 3D mathematical model.

### BRIEF DESCRIPTION OF THE DRAWINGS

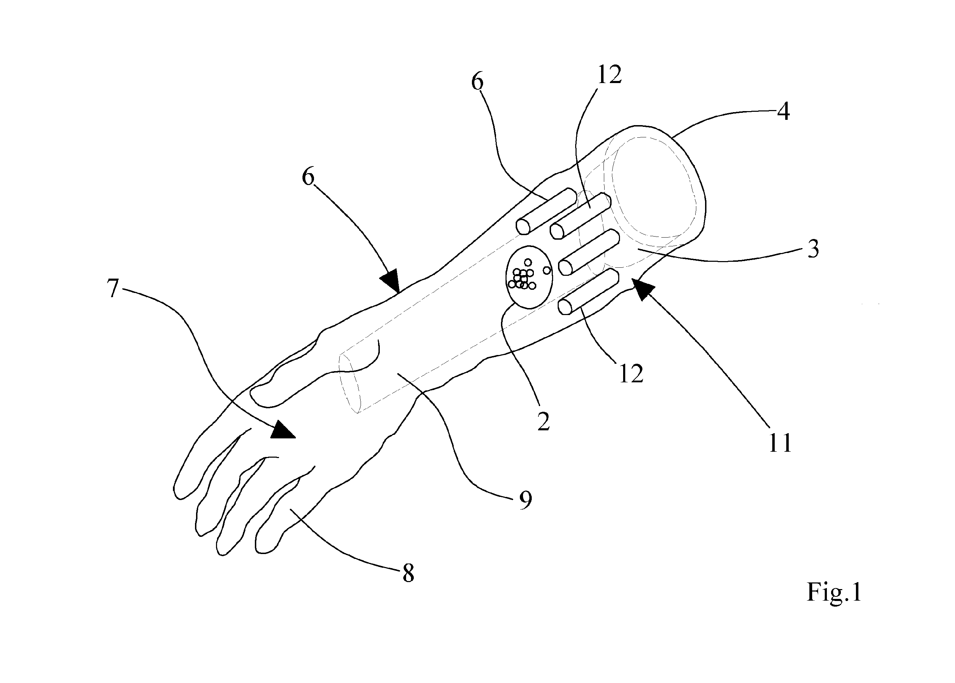

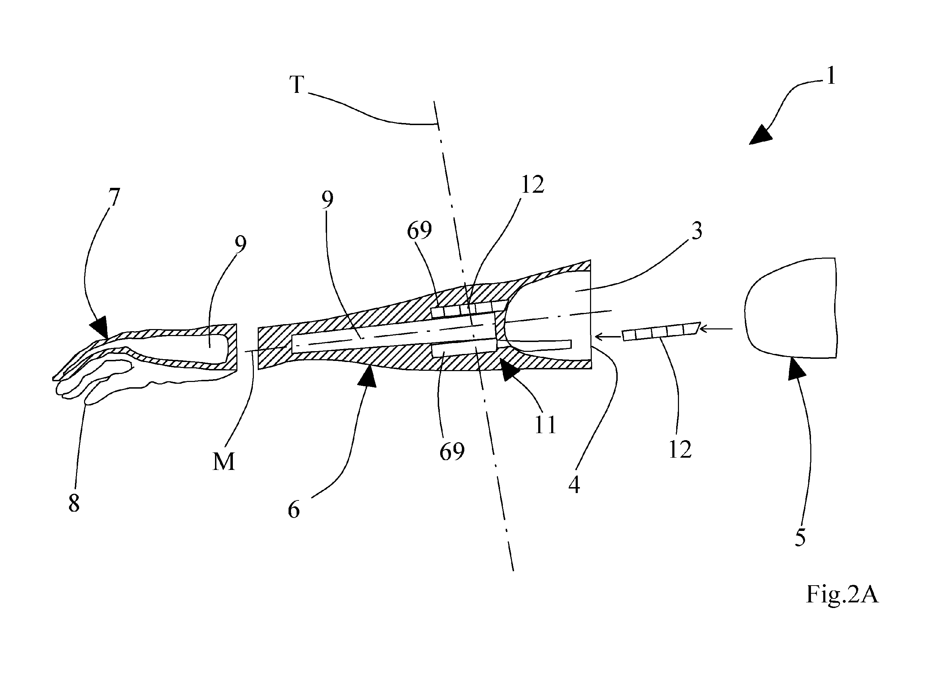

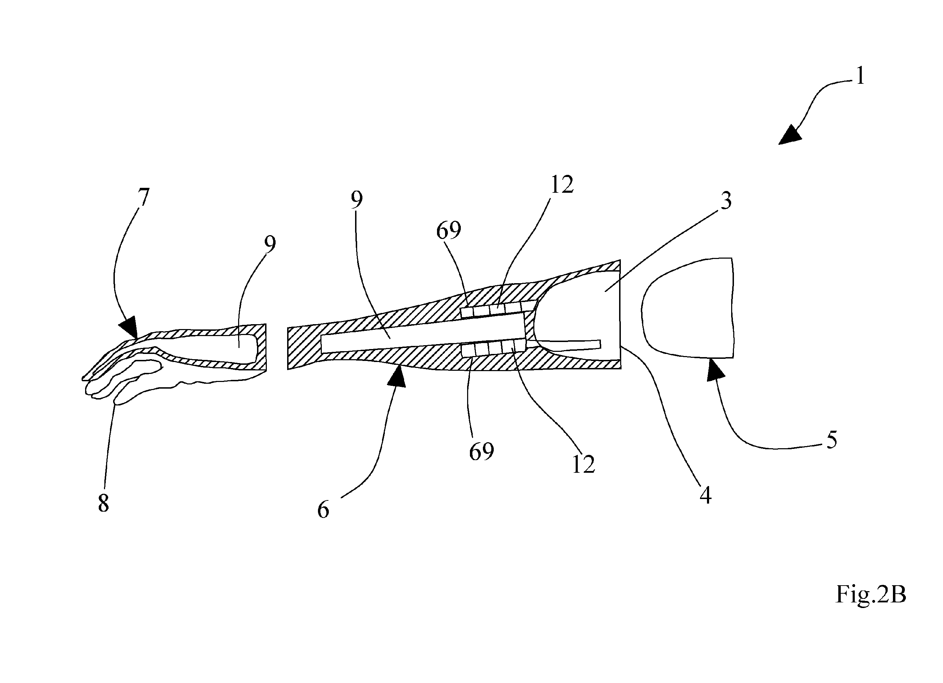

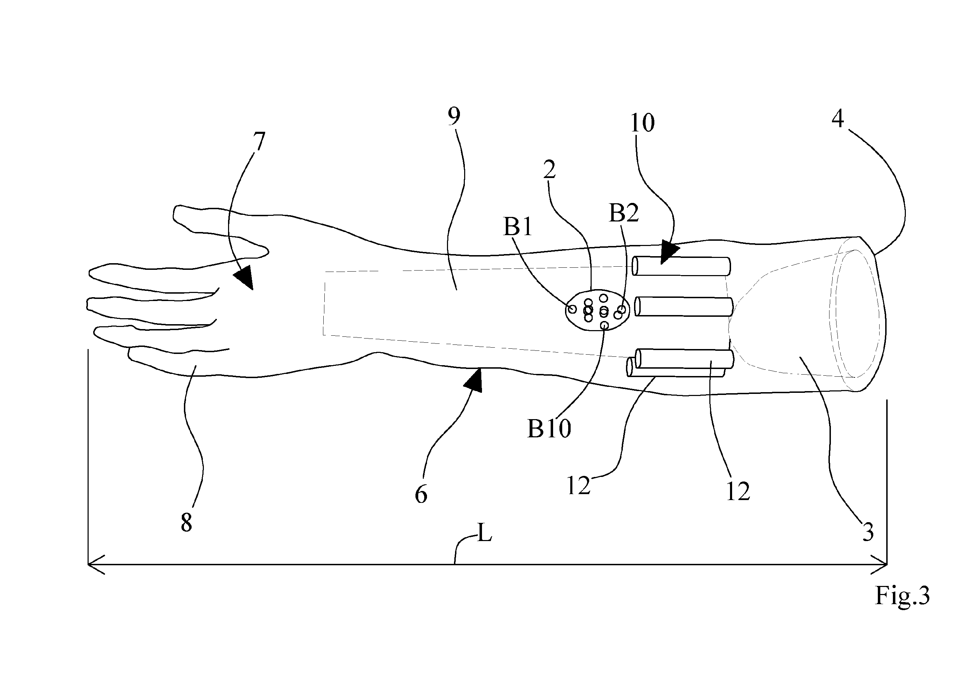

Some embodiments and some aspects of the invention will be described herein below with reference to the accompanying figures of the drawings, provided by way of non-limiting example, in which:
➢ figure 1 is a perspective view of a passive artificial limb;
➢ figure 2A is a section view of the passive artificial limb of figure 1 in a first configuration;
➢ figure 2B is a second view of the passive artificial limb of figure 1 in a second configuration;
➢ figure 3 is a view from above of the passive artificial limb of figure 1;

### DETAILED DESCRIPTION

With reference to the accompanying figures, numeral 1 denotes in its entirety a passive artificial limb. In the following description, the artificial limb will also be defined as a passive prosthesis. The artificial limb is in fact a passive cosmetic prosthesis structure used for example in patients suffering from amelia, ectromelia, hemimelia, peromelia and/or other diseases. The prosthesis is preferably used for unilateral patients (lack of a single upper limb or of only one lower limb), but does not exclude the use for bilateral patients. The artificial limb is used as a substitute for the missing part of the subject while allowing the subject to carry out activities such as using it as a support or a lever for objects.

In the accompanying figures, the passive artificial limb is applied, but not limited to, replacing an upper limb. Aspects of the present invention can also be applied to a prosthesis for the replacement of limbs or portions of the lower limbs. As will be explained in more detail below, the prosthesis of the invention enables varying at least a moment of inertia of the prosthesis with respect to at least one reference axis. The variation of at least a moment of inertia can properly balance the prosthesis so as to help the patient during the rehabilitation phase, as it enables modification of the prosthesis so that it can be modified and adapted to suit the wearer's needs. In a further aspect thereof, the prosthesis 1 allows a balancing of the loads on the stump to be carried out, by varying the weight and/or the position of the centre of gravity of the prosthesis.

In greater detail in reference to the accompanying drawings, the artificial limb 1 comprises a socket 3 arranged on a first end 4 of the limb. The socket 3 exhibits a shape suitable for abutting a stump 5 of a limb and for connecting the stump 5 with the rest of the artificial limb. The socket 3 is customized for each patient since it must enable a perfectly-adhering fit to the stump 5 without causing discomfort and/or pain and/or allergic reactions. To this end, the socket 3 is made of materials such as textile materials, thermoplastic materials, composite materials that are suitable for acting in contact with the patient's stump 5. The socket 3 affects the control of the prosthesis by the patient and can be structurally realized in two different types which differ in terms of the nature of the contact established between the socket and the stump 5. In a first type, called total contact, the socket defines a seating where the stump 5 is directly accommodated. In a second type, an interface is obtained by interposing a "liner" (for example a bag of silicone or an empty bag) 3 between the socket and the stump 5. The artificial limb 1 further comprises a connecting part 6 (for example a prosthetic arm or a forearm), which is interposed between the socket 3 and a terminal part 7. The terminal part may include, for example, one or more passive articulations. The connecting part 6, in accordance with the structure of natural limbs, has an elongate shape. It may be constituted by a solid or a hollow body. In general, the connecting part 6 must have a certain stiffness in order to transfer loads from the terminal part 7 to the socket and then to the stump so as to allow at least a passive functionality of the limb 1. In the accompanying figures, a connecting part 6 is represented, comprising a cavity 9 which extends at least partially along the longitudinal development of the prosthesis. In a non-limiting way, the cavity 9 exhibits a substantially cylindrical shape which extends from the socket 3 to the terminal part 7. Optionally, cavities 9 having different profiles can be made, which adapt to the shape and dimensions of the patient's prosthesis.

The accompanying figures show, but are not limited to, a situation in which the prosthesis exhibits a single cavity 9, but a connecting part 6 can be realised which comprises a plurality of cavities 9 extending in a longitudinal direction and/or in the transversal direction with respect to the prevailing development direction of the artificial limb 1.

The cavity 9 functions as a lightening aid for the artificial limb 1, with the aim of significantly reducing the efforts the patient has to make during the rehabilitation stage, while not affecting the rigidity of the artificial limb 1. For this purpose, in a cross section of a longitudinal development of direction of the connecting part 6, the cavity or cavities are bounded circumferentially and encapsulated by closed walls of the part 6.

Lastly, note that one or more cavities are also present in the terminal part 7, as shown in figure 2A and figure 2B. In this case too the cavities are perimetrally closed by the walls defining the terminal part 7. In addition to the reduction of the efforts required of the user, the lightening of the prosthetic limb enables the centre of gravity of the prosthesis to be neared to the patient's stump 5 so as to ensure greater control and manoeuvrability of the prosthesis.

The connecting part 6 further comprises an adjusting device 10, which enables at least an inertial momentum of the artificial limb 1 to be varied with respect to at least a reference axis. For example, the adjusting device 10 enables the an inertial momentum of the connecting part of the limb 1 to be varied with respect to an axis, for example the transversal axis T (inserted in figure 2A) passing through the proximal end of the limb 1 and transversal to the longitudinal centre line M (inserted in figure 2A) of the artificial limb. In greater detail, the adjusting device 10 is associated with the portion 6 and configured such as to enable the position of the centre of gravity of the limb 1 to be varied within a control volume 2 (see figure 1) delimited axially between a rear section, proximal to the socket 3, and a front section, which is a function of the length of the artificial limb 1. In fact, the front section of the volume 2, which defines the section of maximum excursion of the centre of gravity, is distanced from the socket by at least 1/4 of the length L of the artificial limb 1. In this context, the length of the artificial limb 1 means the extension of the ideal line generated by the union of the geometric centres of gravity of the cross sections 1 of the artificial limb 1. In this regard, note that the length of the prosthesis varies depending on the subject wearing it and the part that the prosthesis must replace. In the case of forearm prostheses for adults, the length L is between 30 and 50 cm; for example, in a case of a forearm prostheses that is 42 cm long, the adjusting device 10 can enable adjustment of the axial position of the centre of gravity along the connecting part 6 within a volume bounded axially between the rear section, next to the socket, and a front section at about 10-11 cm. from the socket. The control volume 2 is also delimited radially, in the sense that the device 10 is also configured to displace the centre of gravity of the artificial limb 1 in a radial direction, even where this movement is relatively small as shown in figure 3 where B1, B2 ... B10 represent some of the positions that the centre of gravity of the limb 1 can assume thanks to the adjusting device 10. In accordance with an embodiment of the invention, the maximum radial excursion of the centre of gravity allowed by the adjusting device 10 is not more than 30% of the radial maximum of the connecting part 6.

As previously mentioned, the adjusting device 10 also enables a variation in weight and/or the position of centre of gravity of the artificial limb 1. In the example described herein, the adjusting device 10 enables the weight and/or the position of the centre of gravity of the connecting part 6 to be varied.

The adjusting device 10 comprises a predetermined number of areas of application 11, arranged on the connecting part 6, each of which is intended to receive one or more respective discrete bodies 12, separate from one another. In the example illustrated in the accompanying figures the discrete bodies 12 are equal in volume, shape and mass. However, it is possible to provide two or more groups of discrete bodies that are different from one another, for example in terms of mass and/or shape and/or volume.

In a non limitative manner, the accompanying figures show areas of application 11 arranged inside the artificial limb, a situation which enables the aesthetic appearance of the passive artificial limb 1 to be maintained unaltered. However, the zones of application 11 can be provided on an external surface of the limb.

The application zones 11 comprise a predetermined number of housing seatings 69 suitable for containing the discrete bodies 12. In the illustrated example each seating is configured such as to accommodate at least a discrete body 12. In an alternative embodiment, the seatings 69 can be configured such as each to receive two or more discrete bodies. The housing seatings 69 are at least partially adaptable to the shape of the discrete bodies 12, establishing a reversible connection between the connecting part 6 and the discrete bodies 12.

As the accompanying figures show, the housing seatings 69 can be arranged in a space extending radially between the cavity 9 and the outer surface of the artificial limb. In particular it can be seen in the accompanying figures that the housing seatings 69 are arranged in an ideal circular crown extending around the cavity 9. The seatings 69 are also angularly equally-distanced at an angular spacing of less than 180°, for example less than 90°, so that four or more locations, and therefore four or more discrete bodies, can be arranged on the circular crown around the cavity 9. The angular spacing depends in general on the number of seats present on the connecting part 6: an increase in the number of housing seatings 69 corresponds to a decrease in the spacing angle. Alternatively or in combination with the arrangement of the seatings 69 according to a circular crown, a predetermined number of housing seatings 69 can be arranged in axially-successive positions, for example along longitudinal lines with respect to the development of the artificial limb 1.

Note that in the illustrated example the housing seatings are opened at the socket, thus defining access openings for insertion of discrete bodies which can then be inserted into the seatings when the artificial limb is separated from the stump. Seatings and discrete bodies can be of prismatic shape, e.g. cylindrical, such as to allow easy insertion and removal by the user. It should also be noted that one or more insert holders may be provided that are removably insertable in the artificial limb so that the user can first load the holder with the number of inserts desired, before coupling the holder to the artificial limb. In this case too, the insert bodies can be inserted into a suitable complementarily-shaped seating having an access opening which for example exclusively faces the socket 3.

From the attached figures it can be noted that regardless of the arrangement of discrete bodies (crown-arranged or in longitudinal alignment) they are arranged internally of a portion of the connecting part 6 which is between the socket 3 and the centre of gravity of the prosthesis. This arrangement will assist the stump 5 in supporting the momentum generated by the terminal part 7 at the point of attachment between 1 artificial limb and the stump 5.

In greater detail, the discrete bodies 12 can be seen to exhibit an insert conformation having a substantially prismatic shape, e.g. cylindrical. However, discrete bodies 12 can be realised having spherical or polyhedral conformation. The inserts are non-limitedly constituted by a single body: alternatively modular inserts might consist of at least two parts.

The discrete bodies have for example a volume of less than 5000 mm³, optionally less than 4000 mm³. The weight of said bodies is for example less than 100g, optionally less than 50g. In an embodiment, the volume of each cylinder-shaped body is 3928 mm³ and the weight of each cylinder is 45g. This is in order to finely adjust the position of centre of gravity of the overall prosthesis so that it is better adapted to the specific load needs of the subject's prosthesis.

In a further aspect, in the case of an artificial forearm, the overall weight of the unloaded artificial limb (devoid of discrete bodies) and the discrete bodies satisfy the following relations:
- ratio of unloaded limb weight and total weight discrete bodies comprised between 1 and 3,
- ratio of total limb weight and weight of each of the discrete bodies of between 5 and 10, preferably between 4 and 8.

The passive artificial limb 1, as already mentioned herein above, includes a terminal part 7 arranged on a second end 8 of the limb artificial 1, from the opposite side to said first end 4.

The terminal part 7 is for example a hand or a prosthetic foot and is the part which should be similar to the morphology of the natural hand or foot. In addition, the terminal part 7 must be light in weight and high in strength. These aspects are particularly relevant because the terminal part 7 is the most exposed to stresses and also determines the momentum the stump 5 is subjected to.

The artificial limb 1 can further comprise a covering glove (not shown in the figures) which exhibits a surface finish capable of conferring a substantially similar appearance to the skin surface of the subject on whom the artificial limb is applied. The glove also has a colour appearance that is substantially similar to the chromatic appearance of the skin of the subject the prosthesis is applied on. The glove is then fitted to the artificial limb 1 and has mainly aesthetic purposes.

The glove is made of plastic material, for example silicone plastic; one or more of the following materials can be used: silicone, PVC; though this is non-limiting.

The above-described artificial limb enables simple modulation of the weight of the limb and the position of the centre of gravity. The insertion and extraction of the discrete bodies one at a time or in a group (if a holder is used) enables the prosthesis to adapt the weight and distribution easily and without any need for third parties to help. The user can simply separate the limb from the stump and insert/remove discrete bodies at will until the desired weight and inertial effect are achieved.

Thanks to the structure of the adjusting device there is no negative impact on the appearance of the artificial limb, and the individual is guaranteed easy use of the limb.

### MANUFACTURING PROCESS.

A manufacturing process of the passive artificial limb 1 is now described.

A first step involves a plotting of the shape of the patient's stump 5. This step can be avoided if, for example, the template is already available or if a standard profile is to be adopted. The step of ascertaining the shape of the stump 5 is useful in modelling a socket 3 for the prosthesis that is as customised as possible to the individual. The profiling of the shape can be achieved, but not limited to, a negative plaster cast of the abutment 5 from which the positive cast of the stump 5 is realised. Using this cast, the socket 3 of the artificial limb 1 can be realised, for example by moulding. The socket profiling stage can alternatively be realised using a three-dimensional scanning process with the creation of a 3D mathematical model that can describe the conformation of the scanned abutment. Also, where a mathematical model of the abutment is to be created, this can be achieved by 3D scanning of the previously-described positive cast.

A second step comprises the definition of the shape of the connecting part 6 and the terminal part 7. This phase may also use standard models or can be obtained by a scanning process of any other 3D scan of the subject's other limb. The scanning process detects a cloud of 3D plotted points of the patient's natural limb, or detects a cloud of points of a real physical model representative of a limb as similar as possible to the missing limb of the patient. Following the acquisition, by means of data processing software, a mathematical model is generated that is representative of a specular shape to that of natural limb of the subject.

A third step involves the construction of the physical model of the artificial limb. This phase may for example be realized by injection moulding using moulds shaped taking into account the cast of the stump and of the shape to be given to the remaining parts of the limb e.g. defined on the basis of the mathematical model in the second step as described above. Alternatively and in a non-limiting manner, the artificial limb 1 can be realised using a rapid prototyping process taking account of the mathematical model of the stump or of the positive mould thereof (first step) and of the mathematical model of the rest of the limb (second step). In particular, if the prosthesis is made of a plastic material, the rapid prototyping process includes the acquiring of a three-dimensional mathematical model as just described and the polymerisation or the selective sintering of liquid and/ or fluid and/or powder material so as to define a body that is identical to the profile of the model.

The prototyping can be accomplished using various techniques such as the Polyjet technique (depositing of layers of liquid sensitive photopolymers hardened by UV rays), Multi Jet Modeling (depositing of layers of thermoplastic resin), Laser Sintering (by means of laser sintering of powders metal or thermoplastic or silica), and so on.

Rapid prototyping techniques allow a wide-ranging flexibility during production and ensure the possibility of realising closed cavities 9. The use of techniques such as injection moulding ensures greater repeatability, but at the price of costs for the mould and need to properly conform the moving parts thereof.

## Claims

1. A passive prosthetic limb, comprising:
at least a socket (3) arranged at a first end (4) of the prosthetic limb (1) and
exhibiting a shape designed to abut a stump (5) of a subject's limb,
at least a terminal part (7), optionally shaped as a hand or a foot, arranged at a second end (8) of the passive prosthetic limb (1) opposite the first end (4),
at least a connecting part (6) interposed between the socket (3) and the terminal part (7),
the connecting part (6) comprising at least an adjusting device (10) configured such as to vary at least a moment of inertia of the passive prosthetic limb (1) with respect to at least a reference axis.

2. The prosthetic limb of claim 1, wherein the adjusting device (10) enables a variation of the weight and/or the position of a centre of gravity of the passive prosthetic limb (1), optionally wherein the adjusting device (10) enables the weight and/or the position of the centre of gravity of the connecting part (6) to be changed.

3. The prosthetic limb of any one of the preceding claims, wherein the adjusting device (10) comprises a predetermined number of application zones (11), arranged on the connecting part (6), each of which is configured to receive one or more respective discrete bodies (12), each of the discrete bodies (12) exhibiting a predetermined dimension and weight, such that addition or removal of one or more of the discrete bodies (12) from the application zone (11) determines a change in weight and/or at least a moment of inertia of the passive prosthetic limb (1), and wherein the application zones (11) comprise a predetermined number of housing seatings (69) destined to contain the discrete bodies (12).

4. The prosthetic limb of claim 3, wherein each of the discrete bodies (12) is conformed as an insert, and is removably insertable in at least one seating of the housing seatings (69), optionally wherein each of the housing seatings (69) is configured such as exclusively to contain the respective discrete body (12).

5. The prosthetic limb of any one of claims 3 or 4, wherein the adjusting device (10) comprises at least a set of discrete bodies (12) that are substantially identical.

6. The prosthetic limb of any one of claims from 3 to 5, wherein the discrete body (12) is a modular insert composed of at least two parts, and/or wherein the device comprises at least a holder insertable removably in the connecting body and configured to carry a plurality of discrete bodies.

7. The prosthetic limb of any one of claims from 3 to 6, wherein the discrete body (12) exhibits a substantially prismatic shape that is complementarily shaped to the respective seating.

8. The prosthetic limb of any one of claims from 3 to 7, wherein the seatings exhibit a respective access opening for insertion of the discrete bodies, each access opening facing towards an outside of the limb at the socket in order to enable insertion of the discrete bodies when the prosthetic limb is separated from the stump, and wherein the seatings extend internally of the limb, in particular internally of the connecting part, remaining accessible exclusively from the respective access openings, the insertion and removal of the discrete bodies not altering the appearance of the lateral surface of the connecting part.

9. The prosthetic limb of any one of the preceding claims, wherein the connecting part (6) exhibits one or more cavities (9) which extend at least partially along the longitudinal development of the connecting part (6), in particular wherein the cavity (9) is a lightening cavity, and wherein at least one of the cavities (9) extends substantially from the socket (3) to the terminal part (7).

10. The prosthetic limb of claim 9, wherein the housing seatings (69) are arranged in a space extending radially between the cavity (9) and an external surface of the connecting part (6), and wherein a predetermined number of the housing seatings (69) are arranged in a circular crown exhibiting an angular spacing of less than 180°, optionally in which the angular spacing is less than 90°, and/or in which a predetermined number of housing seatings (69) are subsequently arranged along longitudinal lines that are substantially parallel with respect to the longitudinal development of the passive prosthetic limb (1).

11. The prosthetic limb of any one of the preceding claims from 2 to 10, wherein the adjusting device (12) is configured such as to vary the position of the centre of gravity of the passive prosthetic limb (1) internally of a control volume (2).

12. The prosthetic limb of the preceding claim, wherein the control volume (2) exhibits a radial size that is at most 30% of a maximum radial size of the prosthetic limb (1), and wherein the control volume (2) exhibits an axial size that is at most 25% of the maximum axial size of the prosthetic limb (1), and wherein the control volume extends between the socket (3) and a maximum axial excursion section of the centre of gravity, the maximum axial excursion section being distant from the socket by a quantity which is at least from ¼ to 1/3 of the length of the prosthetic limb (1).

13. The prosthetic limb of any one of the preceding claims from 3 to 12, wherein the discrete bodies (12) are arranged between the socket (3) and the centre of gravity of the prosthetic limb (1).

14. A process for realising a passive prosthetic limb (1) according to any one of claims from 1 to 13, comprising:
a) detecting a shape of the patient's stump (5)
b) optionally detecting a shape of a healthy limb belonging to the patient,
c) creating a 3D mathematical model of the prosthetic limb on the basis of the detections of point a) and/or point b),
d) realising a real physical model of the prosthetic limb on a basis of the detection of the shape of the stump and the 3D mathematical calculation.

15. The process of the preceding claim, wherein the step of detecting the shape of the patient's stump comprises realising a mould, for example in plaster of Paris, of the patient's stump, and wherein the step of realising the physical model comprises a step of rapid prototyping to reproduce a substantially identical shape of the 3D mathematical model.
